# EUROPÄISCHE PATENTSCHRIFT

(11) **EP 3 778 853 B1**
(45) Veröffentlichungstag und Bekanntmachung des Hinweises auf die Patenterteilung: **06.10.2021**
(21) Anmeldenummer: 20183281.3
(22) Anmeldetag: 30.06.2020
(51) Int. Cl.: C12M 1/00, C12M 1/02

(54) **WASSERBAD ZUM BEFEUCHTEN EINES INNENRAUMS EINES INKUBATORS**
WATER BATH FOR HUMIDIFYING AN INTERIOR OF AN INCUBATOR
BAIN-MARIE PERMETTANT D'HUMIDIFIER UN ESPACE INTÉRIEUR D'UN INCUBATEUR

(30) Priorität: 12.08.2019 DE 102019121639
(43) Veröffentlichungstag der Anmeldung: 17.02.2021
(73) Patentinhaber: Adolf Kühner AG, 4127 Birsfelden (CH)
(72) Erfinder: Bürgin, Timothy, 4414 Füllinsdorf (CH); Schumacher, Mathias, 79539 Lörrach (DE); Knobel, Simon, 4133 Pratteln (CH)
(74) Vertreter: Kohlmann, Kai

(56) Entgegenhaltungen:
- EP-A1- 2 573 162
- EP-A2- 0 808 657
- EP-A2- 1 552 888
- DE-A1-102011 121 019
- DE-A1-102017 118 729
- DE-B3-102017 104 508
- DE-C1- 3 815 528

## Beschreibung

Die Erfindung betrifft ein Wasserbad zum Befeuchten eines Innenraums eines Inkubators.

Durch das Befeuchten des Gasgemisches im Innenraum des Inkubators wird eine Verdunstung von Zellkulturmedium aus einem oder mehreren Gefäßen reduziert und dadurch ein verdunstungsbedingtes Aufkonzentrieren von Stoffen in dem Zellkulturmedium verhindert.

Das Befeuchten begünstigt jedoch die Vermehrung von Mikroorganismen auf Oberflächen im Innenraum des Inkubators, selbst dann wenn diese aus Edelstahl bestehen. Die feuchten und nassen Oberflächen in einem Inkubator sind potentielle Kontaminationsquellen, die die Zellkultur zerstören können. Dies gilt insbesondere für die Oberflächen eines Wasserbades, von dessen Wasseroberfläche temperiertes Wasser unmittelbar in den Innenraum des Inkubators verdampft und das Gasgemisch in dessen Innenraum befeuchtet.

Wasserbäder müssen daher regelmässig und aufwändig manuell gereinigt werden. Dazu muss das Wasserbad entleert, aus dem Inkubator entfernt, mit desinfizierenden Reinigungsmitteln gereinigt und anschliessend mit Wasser gespült werden. Häufig müssen von der Oberfläche des Wasserbads außerdem Ablagerungen, wie beispielsweise Kalkreste, mechanisch entfernt werden.

Die EP 0 913 199 B1 offenbart eine Einrichtung zur Befeuchtung des Nutzraumes eines Klimaschrankes, die zur Verringerung der Kontaminationsgefahr einer optimale Reinigung des Wasserbehälters zulässt und die die Gefahr einer Kondensatbildung außerhalb des Wasserbehälters ausschließt oder zumindest verringert. Der Nutzraum ist als Innengehäuse in Schrägstellung in einem Außengehäuse des Klimaschrankes eingesetzt. Der untere Bereich des Nutzraumes dient dabei zur Aufnahme von Wasser im Sinne einer Wanne mit geneigter Bodenfläche, wobei die Neigung in Richtung der Rückwand des Nutzraumes erfolgt.

Die DE 10 2013 009 136 A1 offenbart eine Inkubationskammer mit einem Feuchtigkeitsreservoir, einer Klimatisierungseinrichtung, die die Einstellung einer gewünschten Temperatur ermöglicht, sowie eine Halterung für Proben. Das Feuchtigkeitsreservoir ist vorzugsweise als Wanne ausgebildet, deren aktive Oberfläche durch eine Filz- oder Schaumstofflage oder andere wasserspeichernde Stoffe vergrößert ist. Die Klimatisierungseinrichtung verfügt über zwei getrennte Klimatisierungszonen, die getrennt voneinander regelbar sind. Hierdurch sollen reproduzierbare Bedingungen in der Inkubationskammer geschaffen werden.

Die EP 0 808 657 B1 offenbart einen von wasserdichten Wänden begrenzten, allseitig geschlossenen Behälter zur Aufnahme von Wasser, der mindestens teilweise aus wasserdampfdurchlässigem, aber gegenüber flüssigem Wasser dichtem Material gebildet ist. Der Behälter dient zur Befeuchtung von Klimaschränken. Um das Kontaminationsrisiko zu minimieren ist der Behälter als flexibler, durch einen Verschluss wiederbefüllbarer Beutel ausgebildet, wobei mindestens der wasserdampfdurchlässige Teil des flexiblen Beutels eine gegenüber einer glatten und strukturierten Oberfläche, vergrößerte Oberfläche aufweist. Das Befüllen des flexiblen Beutels erfolgt bevorzugt außerhalb des Klimaschrankes. Die strukturierte und dadurch vergrößerte Oberfläche wird vorgeschlagen, um die nach dem Stand der Technik üblichen, großen offenen Verdunstungsflächen zu vermeiden, die je länger sie in Funktion sind, als Kontaminationsrisiko angesehen werden. Der Beutel kann in dem Klimaschrank aufgehangen oder in eine Wanne eingelegt werden. Aus der JP 2017 123786 A ist ebenfalls ein Befeuchtungsbeutel für einen Innenraum eines Inkubators bekannt geworden, der vor Gebrauch über eine zu verschweißende Öffnung mit sterilem Wasser befüllt wird, das über eine wasserundurchlässige, jedoch gasdurchlässige Membran in dem Beutel in den Innenraum des Inkubators verdampft. Um eine Verdampfung des Wassers vor dem Gebrauch des Befeuchtungsbeutels zu verhindern, ist die die Membran aufweisende Öffnung in dem Beutel von außen mit einer ablösbaren, selbstklebenden Abdeckung versehen. Der Befeuchtungsbeutel kann in eine in den Boden des Inkubators eingelassene Vertiefung eingelegt werden.

DE102017104508B offenbart einen Inkubator mit einem Wasserbad zum Befeuchten des Innenraums mit einer externen Flüssigkeitsversorgung und -temperierung des Wasserbads. Der Behälter zur Aufnahme des Flüssigkeitsbades ist durch diese Anordnung gut zugänglich und kann gereinigt und/oder entnommen und gereinigt werden. Kontaminationen durch verkeimte Flüssigkeit in dem Behälter lassen sich dadurch vermeiden. insbesondere erlaubt diese offene Anordnung des Behälters eine vorteilhafte Anordnung der Kondensationsoberfläche. EP 1 552 888 A2 offenbart einen an einen Inkubator angrenzenden und durch eine wasserdampfdurchlässige Membrane getrennten Befeuchtungsraum.

Ausgehend von diesem Stand der Technik liegt der Erfindung die Aufgabe zu Grunde, ein Wasserbad zur Verwendung in Inkubatoren zu schaffen, bei dem der Reinigungsaufwand entfällt und das Risiko von Kreuzkontaminationen reduziert ist.

Diese Aufgabe wird durch ein Wasserbad zum Befeuchten eines Innenraums eines Inkubators mit folgenden Merkmalen gelöst
- eine profilierte, zumindest an der Oberseite, vorzugsweise jedoch an den Stirnseiten und der Oberseite offene Aufnahme mit in Längsrichtung verlaufenden Aufnahmeflächen,
- ein vorkonfektioniertes, an der Oberseite offenes Einweg-Gefäß für Flüssigkeit, wobei die Gefäßwände an den Aufnahmeflächen der Aufnahme bündig anliegen,
- Befestigungsmittel zum Fixieren des Einweg-Gefäßes an der Aufnahme und
- eine Flüssigkeitsversorgung eingerichtet zum Befüllen des Einweg-Gefäßes mit Flüssigkeit.

Das an der Oberseite offene Einweg-Gefäß für Flüssigkeit wird nach dem Gebrauch durch ein neues Einweg-Gefäß ersetzt. Dadurch entfallen der Reinigungsaufwand und die Reinigungs-Validierung. Hieraus resultiert eine Zeit- und dadurch Kostenersparnis. Außerdem reduziert das Einweg-Gefäß die Gefahr von Kreuzkontaminationen. Ein weiterer Vorteil besteht darin, dass die Umweltbelastung durch das Einweg-Gefäß in der biopharmazeutischen Produktion kleiner ist, als die Umweltbelastung durch das Reinigen und Sterilisieren eines herkömmlichen Wasserbades aus Edelstahl. Beim Reinigen und Sterilisieren werden grosse Mengen an teilweise erdölbasierten Reinigungsmitteln und viel Energie verbraucht.

Die Aufnahme ist der Bestandteil des Wasserbades, der nach Gebrauch nicht entsorgt wird. Die Aufnahme besteht beispielsweise aus Edelstahl. Sie kann jedoch auch aus anderen Materialien, wie Aluminium oder Kupfer bestehen. Sofern die Aufnahme an den Stirnseiten offen ist, kann das Einweg-Gefäß in Längsrichtung über die Aufnahmeflächen überstehen. Durch den Überstand des Einweg-Gefäßes oder durch eine an die Form der Aufnahme angepasste Ausgestaltung auch der Stirnseiten des Einweg-Gefäßes wird sichergestellt, dass die gesamte Oberfläche der Aufnahmeflächen mit dem Einweg-Gefäß in Kontakt steht. Dies ist insbesondere dann von Bedeutung, wenn die Aufnahmeflächen der Aufnahme beheizt sind. Dadurch wird vermieden, dass Teilflächen der Aufnahmeflächen nicht mit den Gefäßwänden in Kontakt stehen und dadurch ein unerwünschter Wärmeeintrag von den beheizten Aufnahmeflächen in den Innenraum des Inkubators erfolgt.

Das Einweg-Gefäß ist an der Oberseite zum Innenraum des Inkubators offen, so dass die Wasseroberfläche unmittelbar mit dem Gasgemisch im Innenraum in Kontakt gelangt.

Das Einweg-Gefäß wird für eine bestimmte Zeit, z.B. von wenigen Tagen bis zu einigen Wochen gebraucht, danach entsorgt und anschliessend durch ein Neues ersetzt. Das Einweg-Gefäß ist vollständig vorkonfektioniert. Vorkonfektioniert bedeutet, dass das Einweg-Gefäß durch den Anwender ohne weitere Bearbeitung unmittelbar verwendbar ist.

Durch die bündig an den Aufnahmeflächen anliegenden Gefäßwände wird eine Kontamination in Zwischenräumen zwischen der Aufnahme und dem Einweg-Gefäß vermieden.

Die Befestigungsmittel bewirken eine einwandfreie Positionierung des Einweg-Gefäßes an der Aufnahme und einen optimalen Kontakt zwischen den Aufnahmeflächen und den Gefäßwänden.

Die Flüssigkeitsversorgung erlaubt sowohl die Erstbefüllung des Wasserbades, beispielsweise über einen Wasseranschluss des Inkubators, als auch ein Nachfüllen von Flüssigkeit während des Betriebs des Inkubators.

Das Einweg-Gefäß besteht vorzugsweise aus einem flexiblen Material, z.B. aus einer ein- oder mehrschichtigen flexiblen Folie aus thermoplastischem, d.h. schweissbarem Kunststoff. Grundsätzlich kann das Einweg-Gefäß jedoch auch ein starres, an die Aufnahme angepasstes Kunststoff-Formteil sein. Das Einweg-Gefäß kann steril oder nicht steril sein. Die Sterilisation erfolgt beispielsweise im Wege der Ethylenoxid- oder Gammastrahlensterilisation.

Um das Einweg-Gefäß mit möglichst wenig Material und einer geringen Anzahl an Arbeitsschritten kostengünstig herzustellen, wird in einer vorteilhaften Ausgestaltung der Erfindung ein Einweg-Gefäß vorgeschlagen, das eine im wesentlichen rechteckige Folie aufweist, die entlang einer Längsmittellinie gefaltet und an den Stirnseiten ausgehend von der Längsmittellinie zu den Längsrändern der Folie mit einer Schweißnaht versehen ist.

In einer Ausgestaltung der Erfindung ist in eine der beiden Schweißnähte benachbart, d.h. angrenzend zu der Längsmittellinie ein Abfluss-Schlauch mit einem Absperrorgan eingeschweißt. Mit nur zwei Schweißungen lässt sich das Einweg-Gefäß herstellen und zudem stirnseitig der Abfluss-Schlauch bodennah flüssigkeitsleitend an das Gefäß anschließen. Je weniger geschweisst werden muss, umso weniger Arbeitsschritte sind für die Herstellung des Einweg-Gefäßes erforderlich.

Die profilierte Aufnahme weist in vorteilhafter Ausgestaltung der Erfindung einen V-förmigen Querschnitt auf. Das flexible Einweg-Gefäß mit lediglich zwei Schweißnähten nach den Merkmalen des Anspruchs 3 wird von der V-förmigen Aufnahme optimal aufgenommen. Das sich ergebende Dreiecksprofil ist für das Wasserbad ideal, da das Verhältnis von der Flüssigkeitsoberfläche an der Oberseite der Aufnahme zu dem von dem Einweg-Gefäß aufgenommenen Flüssigkeitsvolumen größer ist, als bei einem herkömmlichen Wasserbad mit einem viereckigen Querschnitt. Insbesondere bei beheizten Wasserbädern wird eine große Verdunstungsoberfläche für die Flüssigkeit bei zugleich kleinem Flüssigkeitsvolumen angestrebt, da eine geringere Menge an Flüssigkeit mittels der Heizung temperiert werden muss. Je kleiner das Flüssigkeitsvolumen, desto schneller ist die Flüssigkeit aufgeheizt. Die große Flüssigkeitsoberfläche und das kleine Flüssigkeitsvolumen erhöhen die Verdunstungsgeschwindigkeit und reduzieren die benötigte Zeit, um den Sollwert für die relative Feuchte im Innenraum des Inkubators zu erreichen. Die zum Aufheizen des geringen Flüssigkeitsvolumens erforderliche geringe Heizleistung hat darüber hinaus den Vorteil, dass der teilweise unerwünschte Wärmeeintrag durch das Wasserbad in den Innenraum des Inkubators reduziert wird.

Sofern die im Querschnitt V-förmige Aufnahme als einteiliges, winkliges Profil ausgestaltet ist, bilden die Schenkel des Profils die Aufnahmeflächen. Der in Längsrichtung verlaufende Übergang zwischen den Schenkeln des Profils ist vorzugsweise verrundet, damit sich die Aufnahme problemlos reinigen lässt.

Zur Beheizung der Aufnahmeflächen sind in vorteilhafter Ausgestaltung der Erfindung an den Rückseiten der beiden Aufnahmeflächen Heizelemente angeordnet. Die Heizelemente sind als elektrische Widerstandsheizungen ausgeführt. Die Widerstandsheizungen sind flächig und weisen vorzugsweise die Form einer Matte auf. Die flache Bauweise der Heizelemente ist raumsparend. Die Befestigung an der Rückseite kann beispielsweise durch Kleben erfolgen. Die Heizelemente temperieren indirekt durch die Aufnahmeflächen- und Einweg-Gefäßoberflächen die Flüssigkeit in dem Einweg-Gefäß.

Um einen gleichmäßigen Wärmeeintrag über die Heizelemente in das Einweg-Gefäß zu gewährleisten, erstrecken sich die Heizelemente vorzugsweise über die gesamte Länge der Aufnahme sowie von deren Boden soweit in Richtung der oberen Öffnung der Aufnahme, dass die Heizelemente etwa, vorzugsweise mindestens bis auf Höhe des maximalen Flüssigkeitsstandes in dem in die Aufnahme eingesetzten Einweg-Gefäß reichen. Die Widerstandsheizung temperiert indirekt über die Aufnahmeflächen- und Einweg-Gefäßoberfläche das Wasser im Einweg-Gefäß. Die Widerstandsheizung ist kein zwingender Bestandteil des Wasserbades, wird jedoch in der Regel verbaut.

Um einen direkten Wärmeeintrag in den Innenraum des Inkubators durch die Heizelemente weitgehend zu unterbinden, sind die Heizelemente in einer vorteilhaften Ausgestaltung der Erfindung in einem geschlossenen Gehäuse untergebracht, wobei die Aufnahmeflächen, an deren Rückseiten die Heizelemente befestigt sind, ein Bestandteil des Gehäuses bilden. Die Luft in dem geschlossenen Gehäuse hat dabei eine wärmedämmende Funktion. Zusätzlich können wärmedämmende Materialien in dem Gehäuse angeordnet sein.

Ein Einweg-Gefäß mit den Merkmalen des Anspruchs 3 läuft an den Stirnseiten in den die seitlichen Gefäßwände verbindenden Schweißnähten zusammen. Aufgrund des Überstandes des Einweg-Gefäßes oder durch eine an die Form der Aufnahme angepasste Ausgestaltung auch der Stirnseiten des Einweg-Gefäßes wird sichergestellt, dass die gesamte Oberfläche der Aufnahmeflächen Kontakt mit dem Einweg-Gefäß hat. Ein unmittelbarer Wärmeintrag durch einen Teil der Aufnahmeflächen in den Innenraum wird vermieden.

Die Befestigungsmittel zum Fixieren des Einweg-Gefäßes, insbesondere mit den Merkmalen des Anspruchs 3, umfassen entlang der Längsränder des Einweg-Gefäßes angeordnete Öffnungen und in die Öffnungen eingreifende Noppen, die entlang der Längsränder des Einweg-Gefäßes an der Aufnahme oder dem unterhalb der Aufnahme angeordneten Gehäuse befestigt sind. Die Noppen bestehen vorzugsweise aus Edelstahl. Die Öffnungen an den Längsrändern des Einweg-Gefäßes können im Wege eines kostengünstigen Stanzprozesses als Lochstanzungen eingebracht werden. Eine gute Anformung des flexiblen Einweg-Gefäßes an die Aufnahme ist für den Fall beheizter Aufnahmeflächen für einen optimalen Wärmeeintrag in die Flüssigkeit wichtig. Durch das Befüllen des flexiblen Einweg-Gefäßes mit Flüssigkeit werden die Gefäßwände durch die Gewichtskraft der Flüssigkeit an die Aufnahmeflächen angedrückt. Die mehrfache randnahe Befestigung des flexiblen Einweg-Gefäßes verhindert zudem eine Faltenbildung in der das Gefäß bildenden Folie.

Um das Einweg-Gefäß automatisch mit Flüssigkeit zu befüllen und den Flüssigkeitsstand in dem Einweg-Gefäß zu regeln, weist die Flüssigkeitsversorgung einen Schlauch mit einem Ventil auf, welches abhängig von dem Signal eines Schwimmschalters geöffent und geschlossen wird, wobei der Schwimmkörper des Schwimmschalters derart an der Aufnahme angeordnet ist, dass er auf der Flüssigkeitsoberfläche in dem Einweg-Gefäß schwimmt.

Die Regelung des Flüssigkeitsstandes stellt sicher, dass das Einweg-Gefäß nicht vollständig entleert wird oder überläuft. Die Verwendung eines Schwimmschalters ist kostengünstig und trägt zur Zuverlässigkeit des Wasserbades bei. Der Schlauch der Flüssigkeitsversorgung ist mit einem Flüssigkeitsanschluss im Innenraum des Inkubators verbunden. Der Flüssigkeitsanschluss des Inkubators wird entweder aus einem Wasserkanister, der ausserhalb des Inkubators steht, oder einer Wasserleitung gespeist.

Nachfolgend wird die Erfindung anhand eines Ausführungsbeispiels näher erläutert. Es zeigen
- **Figur 1**: eine Folie zur Herstellung eines Einweg-Gefäßes,
- **Figur 2**: eine perspektivische Ansicht des fertigen Einweg-Gefäßes,
- **Figur 3**: eine Aufsicht auf das Einweg-Gefäß nach Figur 2,
- **Figur 4**: eine perspektivische Ansicht des Einweg-Gefäßes in einer V-förmigen Aufnahme,
- **Figur 5**: eine perspektivische Ansicht eines Wasserbads, teilweise im Schnitt dargestellt,
- **Figur 6**: einen Querschnitt durch das Wasserbad nach Figur 5 sowie
- **Figur 7**: einen Längsschnitt durch eine Stirnseite des Wasserbads nach Figur 5.

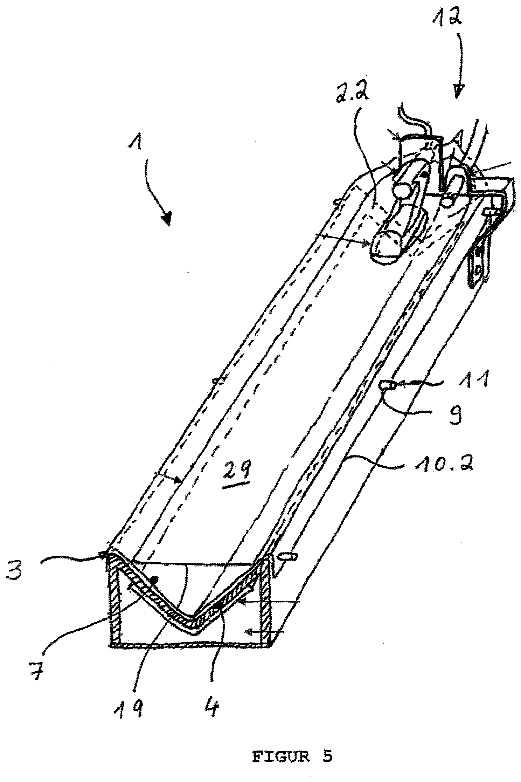

Figuren 5, 6 und 7 zeigen ein erfindungsgemäßes Wasserbad 1 zum Befeuchten eines Innenraums eines nicht dargestellten Inkubators. Das Wasserbad 1 umfasst als wesentliche Bestandteile eine V-förmig profilierte, an den Stirnseiten 2.1, 2.2 und an der Oberseite 3 offene Aufnahme 4 mit zwei in Längsrichtung 5 verlaufenden Aufnahmeflächen 6.1,6.2.

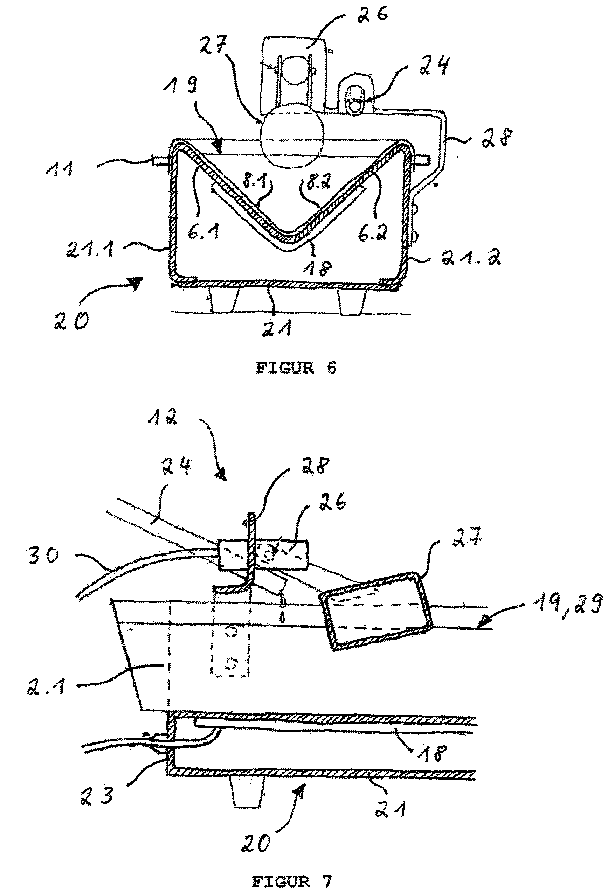

Die Aufnahme 4 nimmt ein vorkonfiguriertes, an der Oberseite 3 offenes, flexibles Einweg-Gefäß 7 für Flüssigkeit, insbesondere steriles Wasser auf, wobei die Gefäßwände 8.1, 8.2 an den Aufnahmeflächen 6.1, 6.2 der Aufnahme 4 bündig anliegen (vgl. Figur 6).

Befestigungsmittel zum Fixieren des Einweg-Gefäßes 7 an der Aufnahme 4 umfassen Öffnungen 9 entlang der Längsränder 10.1, 10.2 des Einweg-Gefäßes 7 und in die Öffnungen 9 eingreifende Noppen 11.

Des Weiteren weist das erfindungsgemäße Wasserbad 1 eine Flüssigkeitsversorgung 12 auf, die zum Befüllen sowie zur Regelung des Flüssigkeitsstandes in dem Einweg-Gefäß 7 eingerichtet ist.

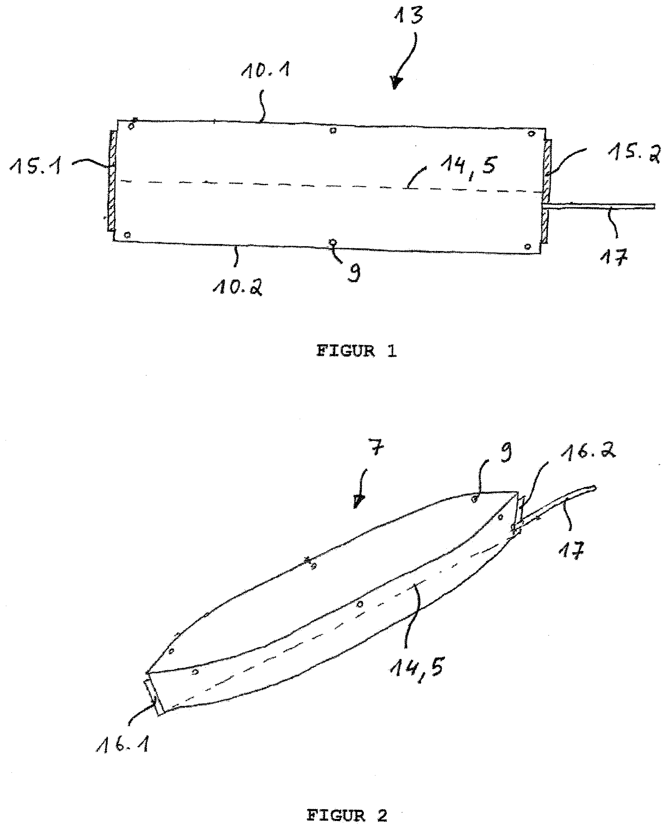

Figur 1 zeigt eine im Wesentlichen rechteckige Folie 13 aus thermoplastischem Kunststoff zur Herstellung des Einweg-Gefäßes 7. Die Folie 13 wird durch die Längsränder 10.1, 10.2 und durch quer zu den Längsrändern 10.1, 10.2 verlaufende stirnseitige Ränder 15.1, 15.2 begrenzt. Die Folie 13 wird entlang einer Längsmittellinie 14 gefaltet und an den stirnseitigen Rändern 15.1, 15.2 jeweils ausgehend von der Längsmittellinie 14 in Richtung der Längsränder 10.1, 10.2 mit einer Schweißnaht 16.1, 16.2 versehen (vgl. Figur 2). In die Schweißnaht 16.2 wird ein Abfluss-Schlauch 17 eingeschweißt. Der Abfluss-Schlauch 17 weist ein nicht dargestelltes Absperrorgan, beispielsweise in Form einer Einweg-Klemme auf, damit das Wasser nur dann aus dem Abfluss-Schlauch 17 austritt, wenn das Einweg-Gefäß 7 entleert werden soll.

Mit lediglich zwei Schweißnähten 16.1, 16.2 und einer Lochstanzung zur Herstellung der Öffnungen 9 lässt sich das Einweg-Gefäß 7 rationell und kostengünstig herstellen.

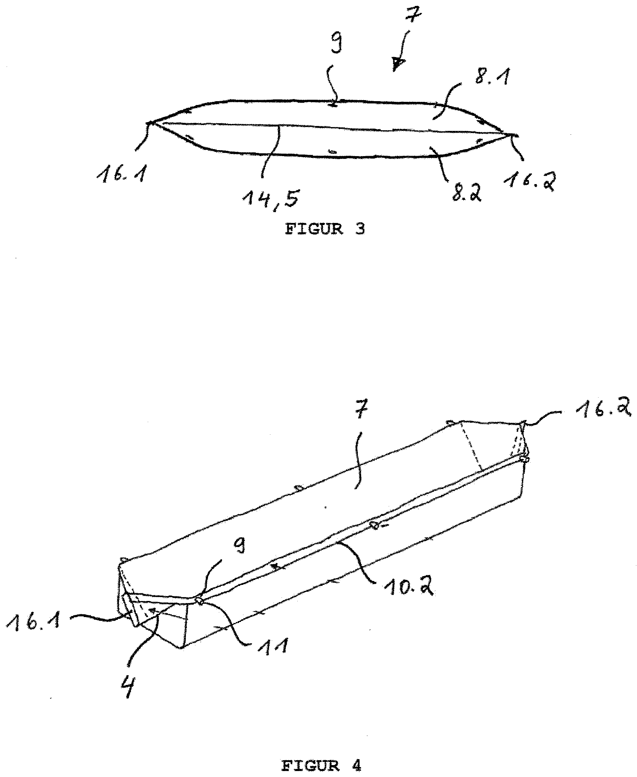

In Figur 4 ist erkennbar, wie das hergestellte Einweg-Gefäß 7 in die Aufnahme 4 eingesetzt und mittels der Noppen 11, die in die Öffnungen 9 entlang der Längsränder 10.1, 10.2 der Folie 13 bzw. des Einweg-Gefäßes 7 eingreifen, an der Aufnahme 4 fixiert wird. Die Fixierung des Einweg-Gefäßes 7 mit einer Vielzahl von Noppen 9 bewirkt, dass das Einweg-Gefäß 7 einwandfrei zu der Aufnahme 4 positioniert wird und mit den Gefäßwänden 8.1, 8.2 faltenfrei an den Aufnahmeflächen 6.1, 6.2 der Aufnahme 4 anliegt. Außerdem begünstigen die Noppen 11 zusammen mit den Löchern 9 eine einfache Montage des Einweg-Gefäßes 7 in der Aufnahme 4.

An den Rückseiten der beiden Aufnahmeflächen 6.1, 6.2 ist ein Heizelement 18 angeordnet (vgl. Figur 6). Das Heizelement 18 ist eine als Matte ausgeführte elektrische Widerstandsheizung, die mit den Rückseiten der Aufnahmeflächen 6.1, 6.2 verklebt ist. Die Matte erstreckt sich nahezu über die gesamte Länge der Aufnahme 4 sowie von deren Boden soweit in Richtung der Oberseite 3 des Wasserbades 1, dass das Heizelement 18 etwa bis zu der Höhe des maximalen Flüssigkeitsstandes 19 in dem Einweg-Gefäß 7 reicht.

Um einen direkten Wärmeintrag in den Innenraum des Inkubators durch das Heizelement 18 weitgehend zu unterbinden, ist das Heizelement 18 in einem geschlossenen Gehäuse 20 untergebracht, das durch einen Gehäuseboden 21, Seitenwände 21.1, 21.2, Stirnwände 23 sowie die V-förmig profilierte Aufnahme 4 begrenzt wird (vgl. Figuren 6, 7). Die Noppen 11 zum Fixieren des Einweg-Gefäßes 7 an der Aufnahme 4 sind an den Seitenwänden 21.1, 21.2 des Gehäuses 20 befestigt.

Die Flüssigkeitsversorgung 12 (vgl. Figur 7) des Wasserbads 1 weist einen Schlauch 24 mit einem elektrisch betätigten Ventil auf. Der Schlauch 24 ist mit einem nicht dargestellten Flüssigkeitsanschluss im Innenraum des Inkubators verbunden, der von einer externen Wasserleitung gespeist wird. Das Ventil wird abhängig von Signal eines Schwimmschalters 26 geöffent und geschlossen. Ein Schwimmkörper 27 des Schwimmschalters 26 ist an einer an dem Gehäuse 20 angeschraubten Halterung 28 drehbeweglich gelagert. Der Schwimmkörper 27 des Schwimmschalters 26 schwimmt auf der Flüssigkeitsoberfläche 29 in dem Einweg-Gefäß 7 und gibt abhängig vom Flüssigkeitsstand in dem Einweg-Gefäß 7 über ein Signalkabel 30 ein Signal an eine Steuerung aus, die das elektrisch betätigte Ventil bei minimalem Flüssigkeitsstand öffnet und bei Erreichen des maximalen Flüssigkeitsstandes 19 schliesst.

Das erfindungsgemäße Wasserbad 1 wird auf dem Boden im Innenraum des Inkubators platziert. Das an der Oberseite offene Einweg-Gefäß 7 befindet sich unterhalb einer Kondensationseinheit, die das Wasser in dem Gasgemisch in dem Inkubator rückkondensiert und dadurch ein Regeln der relativen Feuchte des Gasgemisches ermöglicht. Das rückkondensierte Wasser tropft direkt zurück in das Einweg-Gefäß 7.

Für das Einsetzen und Entfernen des Einweg-Gefäßes 7 in die Aufnahme 4 ist es nicht notwendig, die Aufnahme 4 aus dem Inkubator herauszunehmen.

Für die Entleerung des Einweg-Gefäßes 7 vor dessen Entfernung wird die Klammer des Abfluss-Schlauchs 17 manuell geöffnet. Das ablaufende Wasser kann entweder in einem separaten Gebinde aufgefangen oder durch eine bodennahe Drainage im Inkubator abgelassen werden.

**Bezugszeichenliste**

| | |
|---|---|
| Wasserbad | 1 |
| Stirnseiten | 2.1,2.2 |
| Oberseite | 3 |
| Aufnahme | 4 |
| Längsrichtung | 5 |
| Aufnahmeflächen | 6.1, 6.2 |
| Einweg-Gefäß | 7 |
| Gefäßwände | 8.1,8.2 |
| Öffnungen | 9 |
| Längsränder | 10.1,10.2 |
| Noppen | 11 |
| Flüssigkeitsversorgung | 12 |
| Folie | 13 |
| Längsmittellinie | 14 |
| Stirnseitige Ränder | 15.1, 15.2 |
| Schweißnaht | 16.1,16.2 |
| Abfluss-Schlauch | 17 |
| Heizelement | 18 |
| maximaler Flüssigkeitsstand | 19 |
| Gehäuse | 20 |
| Gehäuseboden | 21 |
| Seitenwände | 22.1,22.2 |
| Stirnwand | 23 |
| Schlauch | 24 |
| Schwimmschalter | 26 |
| Schwimmkörper | 27 |
| Halterung | 28 |
| Flüssigkeitsoberfläche | 29 |
| Signalkabel | 30 |

## Patentansprüche

1. Wasserbad (1) zum Befeuchten eines Innenraums eines Inkubators umfassend
- eine profilierte, zumindest an der Oberseite (3) offene Aufnahme (4) mit in Längsrichtung (5) verlaufenden Aufnahmeflächen (6.1, 6.2),
- ein vorkonfektioniertes, an der Oberseite (3) offenes Einweg-Gefäß (7) für Flüssigkeit, wobei die Gefäßwände (8.1, 8.2) an den Aufnahmeflächen (6.1, 6.2) der Aufnahme (4) bündig anliegen,
- Befestigungsmittel (9, 11) zum Fixieren des Einweg-Gefäßes (7) an der Aufnahme (4) und
- eine Flüssigkeitsversorgung (12) eingerichtet zum Befüllen des Einweg-Gefäßes (7) mit Flüssigkeit.

2. Wasserbad nach Anspruch 1, **dadurch gekennzeichnet, dass** das Einweg-Gefäß (7) aus einem flexiblen Material besteht.

3. Wasserbad nach Anspruch 2, **dadurch gekennzeichnet, dass** das Einweg-Gefäß (7) eine im wesentlichen rechteckige Folie (13) aufweist, die entlang einer Längsmittellinie (14) gefaltet und an den Stirnseiten des Einweg-Gefäßes (7) jeweils ausgehend von der Längsmittellinie (14) zu den Längsrändern (10.1, 10.2) der Folie (13) mit einer Schweißnaht (16.1, 16.2) versehen ist.

4. Wasserbad nach Anspruch 3, **dadurch gekennzeichnet, dass** in eine der beiden Schweißnähte (16.2) ein Abfluss-Schlauch (17) mit einem Absperrorgan eingeschweißt ist.

5. Wasserbad nach einem der Ansprüche 1 bis 4, **dadurch gekennzeichnet, dass** die Aufnahme (4) einen V-förmigen Querschnitt aufweist.

6. Wasserbad nach einem der Ansprüche 1 bis 5, **dadurch gekennzeichnet, dass** die Aufnahmeflächen (6.1, 6.2) der Aufnahme (4) beheizt sind.

7. Wasserbad nach Anspruch 6, **dadurch gekennzeichnet, dass** an der Rückseite der Aufnahmeflächen (6.1, 6.2) mindestens ein Heizelement (18) angeordnet ist.

8. Wasserbad nach Anspruch 7, **dadurch gekennzeichnet, dass** jedes Heizelement (18) in einem geschlossen Gehäuse (20) untergebracht ist und die Aufnahmeflächen (6.1, 6.2) ein Bestandteil des Gehäuses (20) sind.

9. Wasserbad nach einem der Ansprüche 1 bis 8, **dadurch gekennzeichnet, dass** das Einweg-Gefäß (7) an beiden Stirnseiten (2.1, 2.2) der Aufnahme (4) geringfügig über die Aufnahme (4) hinausragt.

10. Wasserbad nach einem der Ansprüche 1 bis 9, **dadurch gekennzeichnet, dass** die Befestigungsmittel zum Fixieren des Einweg-Gefäßes (7) an der Aufnahme (4) entlang der Längsränder (10.1, 10.2) des Einweg-Gefäßes (7) angeordnete Öffnungen (9) und in die Öffnungen (9) eingreifende Noppen (11) umfassen.

11. Wasserbad nach einem der Ansprüche 1 bis 10, **dadurch gekennzeichnet, dass** die Flüssigkeitsversorgung (12) einen Schlauch (24) mit einem Ventil aufweist, welches abhängig von dem Signal eines Schwimmschalters (26) geöffent oder geschlossen wird, wobei ein Schwimmkörper (27) des Schwimmschalters (26) derart angeordnet ist, dass er auf der Flüssigkeitsoberfläche (29) in dem Einweg-Gefäß (7) schwimmt.

12. Wasserbad nach einem der Ansprüche 1 bis 11, **dadurch gekennzeichnet, dass** das Einweg-Gefäß (7) aus thermoplastischem Kunststoff besteht.

13. Wasserbad nach einem der Ansprüche 1 bis 12, **dadurch gekennzeichnet, dass** die profilierte Aufnahme (4) an den Stirnseiten (2.1, 2.2) und der Oberseite (3) offen ist.

## Claims

1. A water bath (1) for humidifying an interior of an incubator, comprising:
- a profiled receiver (4) which is open at least on the upper side (3) having receiver surfaces (6.1, 6.2) running in a longitudinal direction (5),
- a prefabricated disposable vessel (7) for liquid which is open on the upper side (3) wherein the vessel walls (8.1, 8.2) lie flush on the receiver surfaces (6.1, 6.2) of the receiver (4),
- fastening means (9, 11) for fixing the disposable vessel (7) on the receiver (4) and
- a liquid supply (12) adapted for filling the disposable vessel (7) with liquid.

2. The water bath according to Claim 1, **characterized in that** the disposable vessel (7) consists of a flexible material.

3. The water bath according to Claim 2, **characterized in that** the disposable vessel (7) comprises a substantially rectangular film (13) which is folded along a longitudinal centre line (14) and is provided with a weld seam (16.1, 16.2) on the end sides of the disposable vessel (7) in each case starting from the longitudinal centre line (14) to the longitudinal edges (10.1, 10.2) of the film (13).

4. The water bath according to Claim 3, **characterized in that** a drainage tube (17) with a shut-off member is welded into one of the two weld seams (16.2).

5. The water bath according to one of Claims 1 to 4, **characterized in that** the receiver (4) has a V-shaped cross-section.

6. The water bath according to one of Claims 1 to 5, **characterized in that** the receiver surfaces (6.1, 6.2) of the receiver (4) are heated.

7. The water bath according to Claim 6, **characterized in that** at least one heating element (18) is arranged on the rear side of the receiver surfaces (6.1, 6.2).

8. The water bath according to Claim 7, **characterized in that** each heating element (18) is accommodated in a closed housing (20) and the receiver surfaces (6.1, 6.2) are a component of the housing (20).

9. The water bath according to one of Claims 1 to 8, **characterized in that** the disposable vessel (7) protrudes slightly beyond the receiver (4) at both end sides (2.1, 2.2) of the receiver (4).

10. The water bath according to one of Claims 1 to 9, **characterized in that** the fastening means for fixing the disposable vessel (7) on the receiver (4) comprise openings (9) arranged along the longitudinal edges (10.1, 10.2) of the disposable vessel (7) and knobs (11) that engage in the openings (9).

11. The water bath according to one of Claims 1 to 10, **characterized in that** the liquid supply (12) comprises a tube (24) with a valve which is opened or closed depending on the signal of a float switch (26), wherein a float body (27) of the float switch (26) is arranged in such a manner that it floats on the liquid surface (29) in the disposable vessel (7).

12. The water bath according to one of Claims 1 to 11, **characterized in that** the disposable vessel (7) consists of thermoplastic material.

13. The water bath according to one of Claims 1 to 12, **characterized in that** the profiled receiver (4) is open at the end sides (2.1, 2.2) and the upper side (3) .

## Revendications

1. Bain-marie (1), destiné à humidifier un espace intérieur d'un incubateur, comprenant :
- un logement (4) profilé, ouvert au moins sur la face supérieure (3), doté de surfaces de logement (6.1, 6.2) s'écoulant dans la direction longitudinale (5),
- un récipient jetable (7) préfabriqué, ouvert sur la face supérieure (3) destiné à recevoir du liquide, les parois du récipient (8.1, .8.2) affleurant les surfaces de logement (6.1, 6.2) du logement (4),
- des moyens de fixation (9, 11), destinés à fixer le récipient jetable (7) sur le logement (4) et
- une alimentation de liquide (12), aménagée pour remplir le récipient jetable (7) avec du liquide.

2. Bain-marie selon la revendication 1, **caractérisé en ce que** le récipient jetable (7) est constitué d'une matière souple.

3. Bain-marie selon la revendication 2, **caractérisé en ce que** le récipient jetable (7) comporte un film (13) sensiblement rectangulaire, qui est plié le long d'une ligne médiane longitudinale (14) et qui sur les faces frontales du récipient jetable (7) est muni d'un joint de soudure (16 .1, 16 .2) partant respectivement de la ligne médiane longitudinale (14) vers les bords longitudinaux (10.1, 10.2) du film (13).

4. Bain-marie selon la revendication 3, **caractérisé en ce que** dans l'un des deux joints de soudure (16.2) est soudé un flexible d'évacuation (17) doté d'un obturateur.

5. Bain-marie selon l'une quelconque des revendications 1 à 4, **caractérisé en ce que** le logement (4) comporte une section transversale en forme de V.

6. Bain-marie selon l'une quelconque des revendications 1 à 5, **caractérisé en ce que** les surfaces de logement (6.1, 6.2) du logement (4) sont chauffées.

7. Bain-marie selon la revendication 6, **caractérisé en ce que** sur la face arrière des surfaces de logement (6.1, 6.2) est placé au moins un élément chauffant (18).

8. Bain-marie selon la revendication 7, **caractérisé en ce que** chaque élément chauffant (18) est logé dans un boîtier (20) clos et **en ce que** les surfaces de logement (6.1, 6.2) sont un élément constitutif du boîtier (20).

9. Bain-marie selon l'une quelconque des revendications 1 à 8, **caractérisé en ce que** sur les deux faces frontales (2.1, 2.2) du logement (4), le récipient jetable (7) saillit faiblement par-dessus le logement (4) .

10. Bain-marie selon l'une quelconque des revendications 1 à 9, **caractérisé en ce que** les moyens de fixation destinés à fixer le récipient jetable (7) sur le logement (4) comprennent des orifices (9) placés le long des bords longitudinaux (10.1, 10.2) du récipient jetable (7) et des tétons (11) s'engageant dans les orifices (9).

11. Bain-marie selon l'une quelconque des revendications 1 à 10, **caractérisé en ce que** l'alimentation de liquide (12) comporte un flexible (24) doté d'une soupape, qui s'ouvre ou se ferme en fonction du signal d'un interrupteur à flotteur (26), un corps flottant (27) de l'interrupteur à flotteur (26) étant placé de telle sorte qu'il flotte sur la surface du liquide (29) dans le récipient jetable (7).

12. Bain-marie selon l'une quelconque des revendications 1 à 11, **caractérisé en ce que** le récipient jetable (7) est constitué d'une matière thermoplastique.

13. Bain-marie selon l'une quelconque des revendications 1 à 12, **caractérisé en ce que** le logement (4) profilé est ouvert sur les faces frontales (2.1, 2.2) et sur la face supérieure (3).
